# EUROPEAN PATENT APPLICATION

(11) **EP 3 278 719 A1**
(43) Date of publication of application: **07.02.2018**
(21) Application number: 17184109.1
(22) Date of filing: 31.07.2017
(51) Int. Cl.: A61B 1/12, A61B 50/22

(54) **UNIVERSAL ENDOSCOPE DRYING CABINET**

(30) Priority: 01.08.2016 US 201662369644 P
(71) Applicant: Cantel Medical (UK) Limited, Beaconside Stafford ST18 0GA (GB)
(72) Inventor: HOOPER, James, North Somerset, Somerset BS21 7XP (GB); WATTS, Steven, Taunton, Somerset TA3 6TX (GB)
(74) Representative: Jenkins, Peter David

(57) **Abstract**

Embodiments for a universal endoscope drying cabinet are disclosed. In an embodiment, an endoscope drying cabinet comprises: a first compartment configured to receive at least one endoscope; a second compartment and a partition separating the first and second compartments. The partition comprises an aperture and an airflow accelerator configured to generate a first pressure in the first compartment and a second pressure in the second compartment, wherein the first pressure is greater than the second pressure. The endoscope drying cabinet further comprises: a conduit extending through the at least one aperture, wherein a proximal end of the conduit terminates in the first compartment and a distal end of the conduit terminates in the second compartment. Additionally, the endoscope drying cabinet comprises: a distal end coupled to the proximal end of the conduit and a proximal end configured to receive at least two different sizes of endoscope distal tips.

## Description

### TECHNICAL FIELD

Embodiments of the present disclosure relate to endoscopes. More specifically, embodiments of the present disclosure relate to a universal endoscope drying cabinet.

### BACKGROUND

After an endoscope is used, a number of steps are required to reprocess the endoscope, so that the endoscope can be used on another patient. Included in the steps of reprocessing an endoscope is flushing the internal channels of the endoscope using tap and/or filtered water. After which, the endoscope is dried. Drying an endoscope, including drying the internal channels of the endoscope, is desirable because bacteria, such as *Pseudomonas aeruginosa,* have been found in both tap and filtered water and is more likely to multiply in moist environments. Conventional drying cabinets for endoscopes, however, may require different connections than the connections used during other reprocessing steps. Changing the connection to the connection required by the endoscope drying cabinet may re-contaminate the endoscope. Alternatively, if a conventional endoscope drying cabinet requires the same connection as the connections used during other reprocessing steps, the endoscope drying cabinet may only be used with reprocessing systems that use that specific connection. Accordingly, there is a need in the art for improved endoscope drying cabinets.

### SUMMARY

Embodiments of the present disclosure relate to a universal endoscope drying cabinet.

In an example, an endoscope drying cabinet comprises: a first compartment configured to receive at least one endoscope; a second compartment; a partition separating the first and second compartments, wherein the partition comprises at least one aperture and an airflow accelerator configured to generate a first pressure in the first compartment and a second pressure in the second compartment, wherein the first pressure is greater than the second pressure; a conduit extending through the at least one aperture, wherein a proximal end of the conduit terminates in the first compartment and a distal end of the conduit terminates in the second compartment; and a connector comprising: a distal end coupled to the proximal end of the conduit and a proximal end configured to receive at least two different sizes of endoscope distal tips.

In another example, a method for drying an endoscope comprises: recelvmg an endoscope drying cabinet comprising: a first compartment configured to receive at least one endoscope; a second compartment; a partition separating the first and second compartments, wherein the partition comprises at least one aperture and an airflow accelerator configured to generate a first pressure in the first compartment and a second pressure in the second compartment, wherein the first pressure is greater than the second pressure; a conduit extending through the at least one aperture, wherein a proximal end of the conduit terminates in the first compartment and a distal end of the conduit terminates in the second compartment; and a connector comprising: a distal end coupled to the proximal end of the conduit and a proximal end configured to receive at least two different sizes of endoscope distal tips; coupling a distal tip of an endoscope to the proximal end of the connector; and activating the airflow accelerator to generate the first pressure in the first compartment and the second pressure in the second compartment.

In even another example, an endoscope drying cabinet comprising: a first compartment configured to operate at a first pressure; a second compartment configured to operate at a second pressure, wherein the first pressure is greater than the second pressure; a conduit extending between the first and second compartments, wherein a proximal end of the conduit terminates in the first compartment and a distal end of the conduit terminates in the second compartment, wherein the proximal end is configured to receive at least two different sizes of endoscope distal tips.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

FIG. 1 is a block diagram depicting an illustrative universal endoscope drying cabinet, in accordance with embodiments of the present disclosure.
FIGs. 2A-2B are schematic illustrations of an example endoscope drying cabinet, in accordance with embodiments of the present disclosure.
FIG. 3 is a schematic illustration of an example endoscope and carrier, in accordance with embodiments of the present disclosure.
FIGs. 4A-4B are schematic illustrations of a connector for use in the universal endoscope drying cabinets depicting in FIGs. 1 and 2A-2B, in accordance with embodiments of the present disclosure.
FIG. 5 is a flow diagram depicting a method for drying an endoscope using a universal endoscope drying cabinet, in accordance with the embodiments of the disclosure.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

### DETAILED DESCRIPTION

While the disclosed subject matter is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

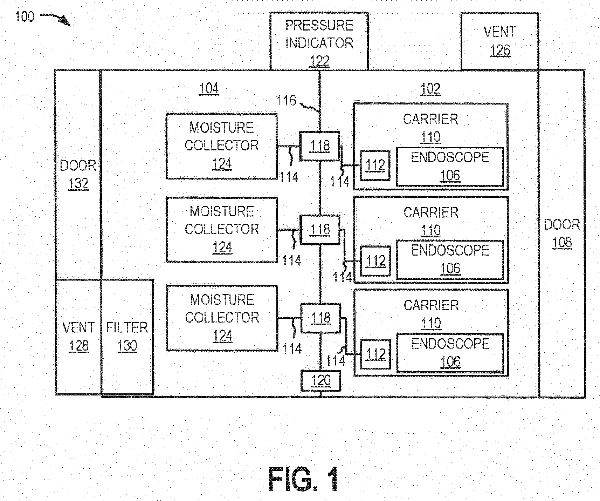

FIG. 1 is a block diagram depicting an illustrative universal endoscope drying cabinet 100, in accordance with embodiments of the present disclosure. The endoscope drying cabinet 100 includes a first compartment 102 and a second compartment 104. The first compartment 102 is configured to receive one or more endoscopes 106, so that the endoscope drying cabinet 100 can dry the endoscopes 106, as described below. In embodiments, the first compartment 102 may include a door 108 for accessing the first compartment 102, so that the endoscopes 106 can be deposited therein. While three endoscopes 106 are depicted in FIG. 1, the first compartment 102 may be configured to accept more or fewer endoscopes 106.

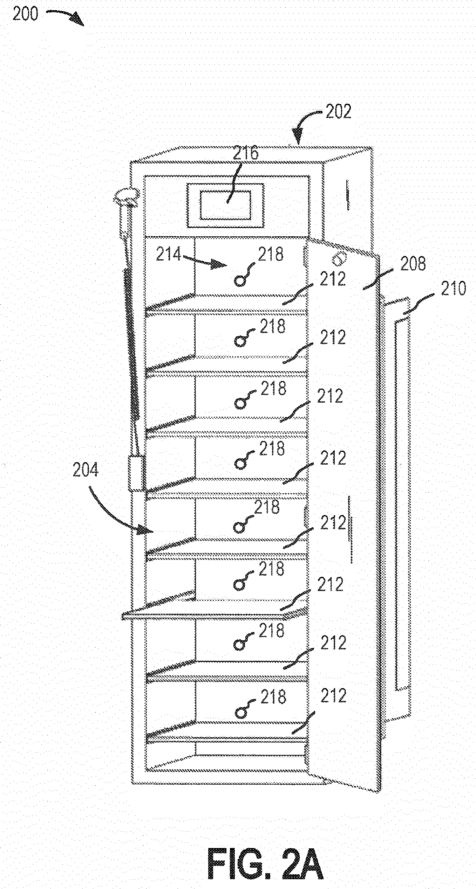

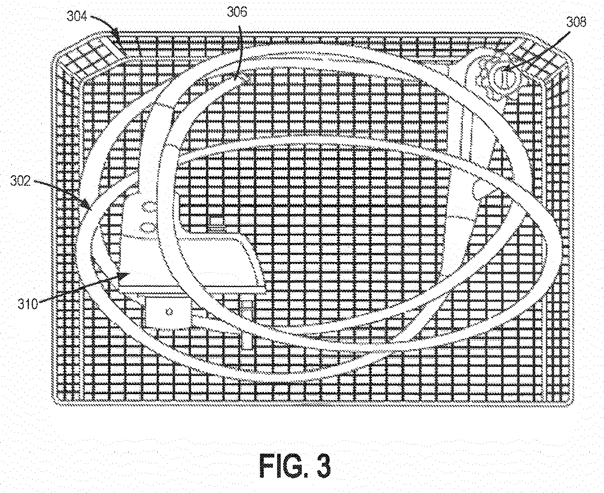

In embodiments, the endoscopes 106 may be placed in one or more carriers 110 that are capable ofbeing received by the first compartment 102. In embodiments, the carrier 110 may be constructed from a mesh so that any liquid transferred from an endoscope 106 to a carrier 110 is not retained by the carrier 110. While each carrier 110 is depicted to hold one endoscope 106, in embodiments, each carrier 110 may be configured to hold more than one endoscope 106. Further, while three carriers 110 are depicted in FIG. 1, in embodiments, more or fewer carriers 110 may be accepted by the first compartment 102. An example carrier 110 is depicted in FIG. 3. In embodiments, the first compartment 102 may include shelves, rails and/or the like to receive and/or separate the carriers 110 from one another. An example of an endoscope drying cabinet 100 that includes shelves is depicted in FIGs. 2A-2B.

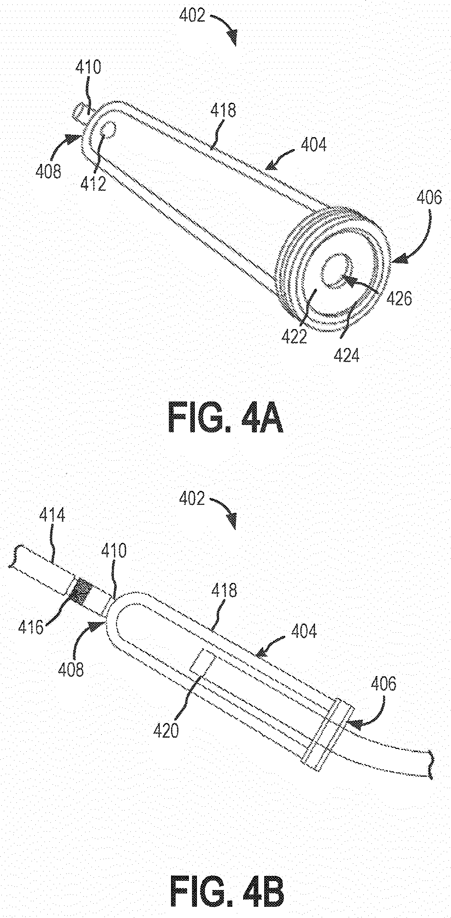

When depositing the endoscope 106 into a carrier 110, a user may couple the endoscope 106 to a connector 112. That is, a proximal end of a connector 112 may be coupled to a distal tip of the insertion tube of an endoscope 106. As described below in relation to FIGs. 4A-4B, the connector 112 is configured to receive endoscopes 106 with different distal tips (e.g., size, shape, etc.).

In embodiments, the distal tip of the connector 112 may be coupled to a proximal end of conduit 114. By coupling the distal tip of the connector 112 to a proximal end of the conduit 114, a suctioning force is applied to the channels of the endoscope 106, due to the pressure differential between the first and second compartments 102, 104, as described below. The suctioning force may aid in the drying of the channels of the endoscope 106. In embodiments, the conduit 114 may be tubing, hosing and/or other types of conduits that are capable of providing a substantially airtight conduit and may, for example, be made from metal, plastic, rubber and/or the like. The distal tip of the connector 112 may be coupled to a proximal end of the conduit 114 via an interference fit, a transition fit or a clearance fit. Additionally or alternatively to providing an interference fit, a transition fit or a clearance fit, the distal tip of the connecter 112 may be coupled to a proximal end of the conduit 114 using an attachment mechanism, e.g., a clamping mechanism. The interference fit, transition fit, clearance fit and/or the clamping mechanism may provide a substantially airtight seal between the distal tip of the connector 112 and the proximal end of the conduit 114. Alternatively, in embodiments, the connector 112 may be incorporated into the conduit 114 and, therefore, the connector 112 and the conduit 114 may be a single unitary structure. While the connectors 112 are shown as being disposed in the carrier 110, in embodiments, the connectors 112 may be located outside of the carrier. Further, while three connectors 112 are shown, more or fewer connectors 112 may be used with the endoscope drying cabinet 100.

In embodiments, the second compartment 104 is separated from the first compartment 102 by a partition 116. The partition 116 is located between the first and second compartments 102, 104, so that the first and second compartments 102, 104 can have different pressures, as described below.

In embodiments, the partition 116 may include one or more apertures 118. The apertures 118 may be configured so that the conduit 114 is capable of extending through the apertures 118. In embodiments, the aperture 118 may surround the conduit 114 to provide a clearance fit, a transition fit or an interference fit. In embodiments, the aperture 118 may surround the conduit 114 to provide a substantially airtight fit around the conduit 114. In embodiments, a proximal end of the conduit 114 may terminate in the first compartment 102 and a distal end of the conduit 114 may terminate in the aperture 118 and/or second compartment 104. Further, as explained above, the proximal end of the conduit may be coupled to the distal end of the connector 112.

In embodiments, the endoscope drying cabinet 100 may include an airflow accelerator 120 that is capable of accelerating air from the second compartment 104 to the first compartment 102. Alternatively, the airflow accelerator 120 may accelerate air from outside the endoscope drying cabinet 100 into the first compartment 102. In embodiments, the airflow accelerator 120 may be capable of operating at different speeds. As such, in embodiments, the pressure differential between the first and second compartments 102, 104 may vary depending on the speed of the airflow accelerator 120. In embodiments, the airflow accelerator 120 may be incorporated into the partition 116. Additionally or alternatively, in embodiments, the airflow accelerator 120 may include a rotor that operates as a fan. Additionally or alternatively, the airflow accelerator 120 may be an external compressed air supply.

To determine the pressure differential between the first and second compartments 102, 104, the endoscope drying cabinet 100 may include a pressure indicator 122. In embodiments, the pressure indicator 122 and the airflow accelerator 120 may be incorporated into a feedback loop, so that a specific pressure difference between the first and second compartments 102, 104 is obtained and/or maintained. In embodiments, the pressure indicator 122 may include a user interface, so that a user can set a specific pressure differential between the first and second compartments 102, 104. In response, the airflow accelerator 120 may operate so that selected pressure is obtained and/or maintained. In embodiments, a user may also set a specific amount of time that the pressure differential is to be maintained by the endoscope drying cabinet 100. Additionally or alternatively, a user interface of the pressure indicator 122 may include the current pressure difference between the first and second compartments 102, 104, so that a user is able to obtain real-time readings of the pressure differences.

Due to a distal tip of the conduit 114 terminating in the second compartment 104 and the proximal end terminating in the first compartment 102 and being coupled to a distal tip of the insertion tube of an endoscope, moisture in the channels of an endoscope 106 may be suctioned out due to the pressure differential that is created between the first and second compartment 102, 104. As such, in embodiments, the second compartment 104 may include a moisture collector 124 to collect any moisture that is suctioned out of one or more channels of an endoscope 106. The moisture collector 124 may include any kind of vessel that is configured to collect and retain moisture (e.g., a bucket). In embodiments, a separate respective moisture collector 124 for each endoscope 106 may be included in the endoscope drying cabinet 100 or a single moisture collector 124 may be used to collect moisture that is suctioned from all the endoscopes 106. Alternatively, more or fewer moisture collectors 124 may be included in the endoscope drying cabinet 100.

In embodiments, the endoscope drying cabinet 100 may include one or more vents 126, 128 that allow air to flow in and out of the endoscope drying cabinet 100. That is, in embodiments, the first compartment 102 may include a vent 126 that allows air to flow out of the first compartment 102. Additionally or alternatively, the second compartment 104 may include a vent 128 that allows air to flow into the second compartment 104. In embodiments, the vent 126 may be configured to restrict the amount of air that flows out of the first compartment 102, so that the amount of air that flows out of the first compartment 102 is less than the amount of air that is being accelerated into the first compartment 102 by the airflow accelerator 120 when the airflow accelerator 120 is in use. Additionally or alternatively, the vent 128 may be configured to restrict the amount of air that flows into the second compartment 104, so that the amount of air that flows into the first second compartment 104 is less than the amount of air that is accelerated out of the second compartment 104 by the airflow accelerator 120 when the airflow accelerator 120 is in use. Alternatively, in embodiments, the vents 126, 128 may be closed during operation of the airflow accelerator 120, opened after the airflow accelerator 120 is no longer accelerating air from the second compartment 104 to the first compartment 102 and/or opened after the endoscope drying cabinet 100 has finished drying the endoscopes 106, so that the pressure in the endoscope drying cabinet 100 and between the first and second compartments 102, 104 can be normalized to the pressure outside of the endoscope drying cabinet 100.

In embodiments, a filter 130 (e.g., a high- efficiency particulate arrestance (HEPA) filter) may be integrated into the vent 128 and/or coupled to the vent 128, so that air flowing into the second compartment 104 is filtered before entering the second compartment 104. Examples of air filters may include, but are not limited to, 0.5 micron filters, 0.2 micron filters 0.1 micron filters, 0.05 microns filters, 0.01 micron filters and/or the like. In embodiments, the second compartment 104 may also include a door 132 for accessing the second compartment 104.

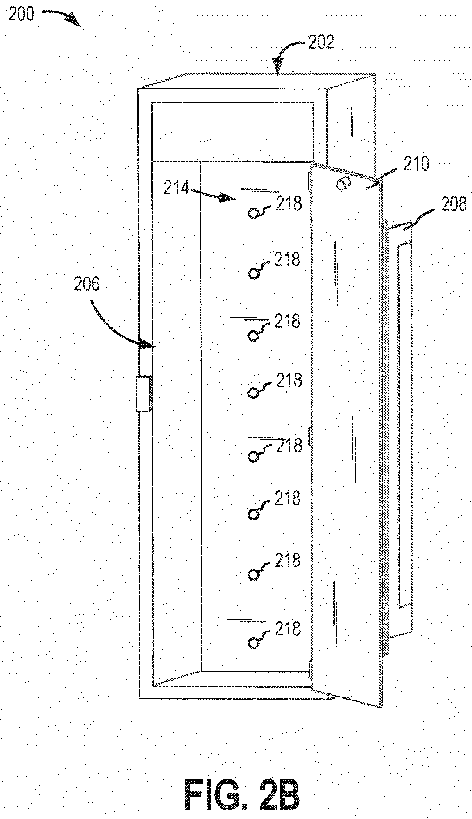

FIGs. 2A-2B are schematic illustrations of an example endoscope drying cabinet 200, in accordance with embodiments of the present disclosure. FIG. 2A depicts a front perspective view of the endoscope drying cabinet 200 and FIG. 2B depicts a back perspective view of the endoscope drying cabinet 200. The endoscope drying cabinet 200 is depicted without any endoscopes, carriers, connectors, conduits and moisture collectors. However, in embodiments, one or more endoscopes (e.g., the endoscopes 106 and/or the endoscope 302 depicted in FIGs. 1 and 3, respectively), one or more carriers (e.g., the carriers 110 and/or the carrier 304, depicted in FIGs. 1 and 3, respectively), one or more connectors (e.g., the connector 112 and/or the connector 402 depicted in FIGs. 1, 4B, respectively), one or more conduits (e.g., the conduits 114) and one or more moisture collectors (e.g., the moisture collectors 124 depicted in FIG. 1) may be incorporated into the endoscope drying cabinet 200.

As shown, the endoscope drying cabinet 200 includes a housing 202, a first compartment 204 and a second compartment 206. In embodiments, the first and second compartments 204, 206 may have some or all of the same characteristics as the first and second compartments 102, 104, respectively, ofFIG. 1. For example, the first and second compartments 204, 206 may include doors 208, 210 for accessing the first and second compartments 204, 206, respectively. As another example, the first compartment 204 may include shelves 212 for receiving endoscopes and/or carriers. In embodiments, the shelves 212 may slide in and out independently and/or simultaneously to facilitate placing an endoscope, a carrier or both on the shelves 212.

The endoscope drying cabinet 200 also includes a partition 214 that separates the first and second compartments 204, 206. In embodiments, the partition 214 may have some or all of the same characteristics as the partition 116 depicted in FIG. 1. For example, the partition 214 may facilitate the first and second compartments 204, 206 operating at different pressures. That is, in embodiments, the first compartment 204 may operate at a higher pressure than the second compartment 206. In embodiments, the pressure differential between the first and second compartments 204, 206 may be facilitated by an airflow accelerator (not shown). For example, an airflow accelerator the same as, or similar to, the airflow accelerator 120 depicted in FIG. 1 may facilitate the pressure differential between the first and second compartments 204, 206.

To determine and/or control the pressure differential between the first and second compartments 204, 206, the endoscope drying cabinet 200 may include a pressure indicator 216. In embodiments, the pressure indicator 216 may have some or all of the same characteristics as the pressure indicator 122 depicted in FIG. 1. For example, in embodiments, the pressure indicator 216 and the airflow accelerator (not shown) may be incorporated into a feedback loop, so that a specific pressure difference between the first and second compartments 204, 206 is obtained and/or maintained. As another example, in embodiments, the pressure indicator 216 may include a user interface, so that a user can set a specific pressure differential between the first and second compartments 204, 206 and/or a user may set a specific amount of time that the pressure differential between the first and second compartments 204, 206 is to be maintained. As even another example, the pressure indicator 216 may display a current pressure of the first and/or second compartments 204, 206 in order to provide a user real-time readings of current pressures.

In embodiments, the endoscope drying cabinet 200 may include apertures 218 that connect the first and second compartments 204, 206. In embodiments, a conduit may extend through a respective aperture 218. As such, when coupled to a distal tip of an insertion tube of an endoscope is coupled to the conduit, a suctioning force may be applied to the channels of the endoscope 106 and facilitate drying the channels of the endoscope. In embodiments, the apertures 218 may have some or all of the same characteristics as the apertures 118 depicted in FIG. 1.

FIG. 3 is a schematic illustration of an example endoscope 302 and carrier 304, in accordance with embodiments of the present disclosure. In embodiments, the endoscope 302 is received by the carrier 304 and the carrier 304 is placed into a first compartment (e.g., the first compartment 102 and/or the first compartment 204 of FIGs. 1 and 2A, respectively) to dry the endoscope. In embodiments, the endoscope 302 may have some or all of the same functionality as an endoscope 106 depicted in FIG. 1. For example, the endoscope 302 includes a distal tip 306 that may be coupled to a connector (e.g., the connectors 112, 402 depicted in FIGs. 1 and 4A-4B, respectively). In embodiments, the endoscope 302 may also include a biopsy port, an air-water valve, a suction valve, a leak test port, and an elevator port in the control body 308 of the endoscope 302. In embodiments, the endoscope 302 may also include a suction port, a water port, an air port, an auxiliary water port, and an auxiliary air port in the light guide connector portion 310 of the endoscope 302. These features are generally known and, therefore, their functions are not discussed further herein.

In embodiments, the biopsy port, the air-water valve and the suction valve in the control body 308 may be capped, for example, by placing a stopper therein when the endoscope 302 is ready to be dried in the endoscope drying cabinet (e.g., the endoscope drying cabinet 100 and/or the endoscope drying cabinet 200 depicted in FIGs. 1 and 2A-2B, respectively). In embodiments, however, the elevator port in the control body 308 and the suction port, the water port, the air port, the auxiliary air port and the auxiliary water port in the light guide connector portion 310 may be left uncapped. As such, when the endoscope 302 is placed in the endoscope drying cabinet and the distal tip 306 of the insertion tube of the endoscope is coupled to a connector, air and/or moisture may flow into the elevator port, the suction port, the water port, the air port, the auxiliary air port and the auxiliary water port, through the channels of the endoscope 302, into the connector/conduit (e.g., the conduits 114 and/or the conduit 414 depicted in FIG. 1 and FIG. 4B, respectively) and, thereafter, into the moisture collector (e.g., the moisture collectors 124 depicted in FIG. 1) due to the pressure differential between the first and second compartments of the endoscope drying cabinet.

In embodiments, the carrier 304 may have some or all of the same functionality as the carrier 110 discussed in relation to FIG. 1 above. For example, as shown, the carrier 304 may be comprised of a mesh, so that any liquid transferred from the endoscope 302. The mesh may be made of metal, plastic, rubber and/or any other materials capable of receiving and retaining the endoscope 302.

FIGs. 4A-4B are schematic illustrations of a connector 402 for use in the universal endoscope drying cabinets depicting in FIGs. 1 and 2A-2B, in accordance with embodiments of the present disclosure. FIG. 4A depicts a perspective view of the connector 402 and FIG. 4B shows a side view of the connector 402. In embodiments, the connector 402 includes a housing 404, a proximal end 406 and a distal end 408. The housing 404 may be formed of glass, plastic, rubber and/or any other material that does not substantially leak air and/or liquids.

The distal end 408 of the connector 402 may have protrusion 410 projecting from an aperture 412 in the distal end 408. The protrusion 410 may have a cross-sectional area and/or shape capable ofbeing coupled to a conduit 414. As such, liquid and/or gas internal to the housing 404 may be in fluid communication with the conduit 414. In embodiments, the conduit 414 may have some or all of the same characteristics as the conduit 114 depicted in FIG. 1. For example, the protrusion 410 may form an interference fit with the conduit 414. Alternatively, the conduit 414 and protrusion 410 may form a transition fit or clearance fit. Additionally or alternatively to forming an interference fit, a transition fit or a clearance fit, a clamping mechanism 416 may be used to secure the conduit 414 to the protrusion 410. Alternatively, instead of protrusion 410, the connector 402 may only include an aperture 412 that is configured to receive the conduit 414. In these embodiments, the aperture 412 and the conduit 414 may form an interference fit, a transition fit or a clearance fit.

In embodiments, the diameter of the housing 404 may increase from the distal end 408 to a central portion 418 of the connector 402. The central portion 418 of the housing 404 may have a cross-sectional area and/or shape that will allow different sized distal tips 420 of endoscopes to fit therein. For example, the central portion 418 may have a cross-sectional area and/or shape that allows the largest manufactured distal tip of an endoscope to fit therein.

In embodiments, the diameter of the housing 404 may increase near the proximal end 406. In embodiments, a larger diameter near the proximal end 406 may facilitate inserting an elastic member 422 therein. The elastic member 422 may be inserted into the housing 404 up to the central portion 418 where the diameter transitions from the smaller diameter of the central portion 418 to the larger diameter of the proximal end 406. In embodiments, the transition in diameter may provide a stopping mechanism so that the elastic member 422 does not slide further into the central portion 418. In embodiments, the proximal end 406 may also include an edge 424 that, once the elastic member 422 is inserted into the housing 404, provides a retaining force on the elastic member 422 that may prevent the elastic member 422 from being pulled out of the housing 404 when a distal tip of an endoscope is being decoupled from the connector 402, as described below. Additionally or alternatively, an adhesive may be applied to the outer rim of the elastic member 422, so that the elastic member 422 is retained within the housing 404. Alternatively, the elastic member 422 and the housing 404 may be integrally formed and, therefore, may be a unitary component. In embodiments, the elastic member may be formed of any type of substantially elastic material that is capable of substantially returning to its original size and shape after being stretched from its original size to+/- 10%, 20%, 30%, 40%, 50% and/or the like greater than its original size. For example, the elastic member 422 may be formed of a rubber material.

The elastic member 422 also includes an aperture 426. In embodiments, a distal tip 420 of an endoscope is inserted into and through the aperture 426 before drying the endoscope with the endoscope drying cabinet. The aperture 426 may be have a cross-sectional size and shape that is configured to receive, without stretching, a specific size distal tip of an endoscope. Additionally, however, the elastic member 422 may stretch so that the aperture 426 is capable of receiving other sized and shaped distal tips of endoscopes. For example, the aperture 426 may stretch to+/- 10%, 20%, 30%, 40%, 50% and/or the like greater than its original size. After the endoscope drying cabinet has finished drying the endoscope, the distal tip 420 of the endoscope may be withdrawn from the housing 404 and the aperture 426 may return to substantially its original size. In embodiments, the elastic member 422 may include slits to facilitate the insertion of different sized distal tips 420 of endoscopes.

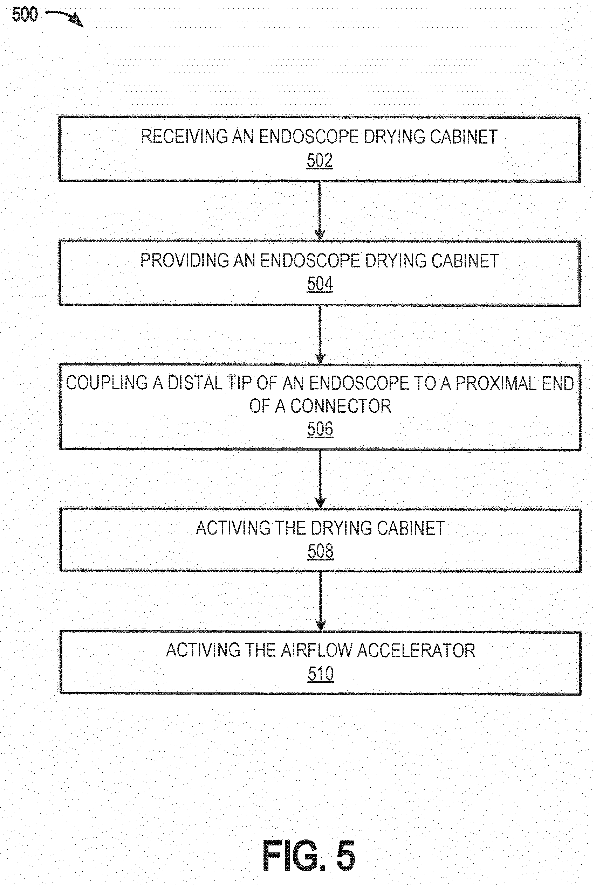

FIG. 5 is a flow diagram depicting a method 500 for drying an endoscope using a universal endoscope drying cabinet, in accordance with the embodiments of the disclosure. In embodiments, the method 500 includes receiving an endoscope drying cabinet (block 502). Additionally or alternatively, the method 500 may include providing the endoscope drying cabinet (block 504). In embodiments, the endoscope drying cabinet may have some or all of the same functionality as the endoscope drying cabinet 100 depicted in FIG. 1 and/or the endoscope drying cabinet 200 depicted in FIGs. 2A-2B. For example, the endoscope drying cabinet may include a first compartment (e.g., the first compartment 102 and/or the first compartment 204 depicted in FIGs. 1 and 2A, respectively) that is configured to receive at least one endoscope (e.g., the endoscope 106 and/or the endoscope 302 depicted in FIGs. 1 and 3, respectively). The endoscope drying cabinet may also include a second compartment (e.g., the second compartment 104 and/or the second compartment 206 depicted in FIGs. 1 and 2B, respectively) and a partition (e.g., the partition 116 and/or the partition 214 depicted in FIGs. 1 and 2A-2B, respectively) separating the first and second compartments. The partition may include at least one aperture (e.g., the aperture 118 and/or the aperture 218 depicted in FIGs. 1 and 2A-2B, respectively) and an airflow accelerator (e.g., the airflow accelerator 120 depicted in FIG. 1) configured to generate a first pressure in the first compartment and a second pressure in the second compartment, wherein the first pressure is greater than the second pressure. Further, in embodiments, the endoscope drying cabinet may include a conduit (e.g., the conduit 114 depicted in FIG. 1 and/or the conduit 414 depicted in FIG. 4B) extending through the at least one aperture, wherein a proximal end of the conduit terminates in the first compartment and a distal end of the conduit terminates in the second compartment. A connector (e.g., the connector 112 and/or the connector 402 depicted in FIGs. 1 and 4A-4B, respectively) may also be used with and/or included in the endoscope drying cabinet. The connector includes a distal end (e.g., the distal end 408 depicted in FIGs. 4A-4B) that may be coupled to the proximal end of the conduit and a proximal end (e.g., the proximal end 406 depicted in FIGs. 4A-4B) that may be configured to receive at least two different sizes of endoscope distal tips.

In embodiments, the method 500 may include coupling a distal tip (e.g., the distal tip 420 depicted in FIG. 4B) of an endoscope to a proximal end of a connector (block 506). In embodiments, the connector may have some or all of the same functionality as the connector 112 and/or the connector 402 depicted in FIGs. 1 and 4, respectively. For example, the connector may include an elastic member (e.g., the elastic member 422 depicted in FIG. 4A) that is capable of receiving different sized distal tips of an endoscope. In embodiments, coupling the distal tip of an endoscope to a proximal end of a connector may include inserting the distal tip of the endoscope through an aperture (e.g., the aperture 426 depicted in FIG. 4A) of an elastic member of the connector.

In embodiments, the method 500 may include activating the endoscope drying cabinet (block 508). In embodiments, activating the endoscope drying cabinet may include any of the functionality of the endoscope drying cabinet described above in relation to FIG. 1. For example, activating the endoscope drying cabinet may include activing an airflow accelerator included in the endoscope drying cabinet until a pressure differential between the first and second compartments is achieved (block 510).

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof

## Claims

1. An endoscope drying cabinet comprising:
a first compartment configured to receive at least one endoscope;
a second compartment;
a partition separating the first and second compartments, wherein the partition comprises at least one aperture and an airflow accelerator configured to generate a first pressure in the first compartment and a second pressure in the second compartment, wherein the first pressure is greater than the second pressure;
a conduit extending through the at least one aperture, wherein a proximal end of the conduit terminates in the first compartment and a distal end of the conduit terminates in the second compartment; and
a connector comprising: a distal end coupled to the proximal end of the conduit and a proximal end configured to receive at least two different sizes of endoscope distal tips.

2. The endoscope drying cabinet of claim 1, wherein the proximal end of the connector comprises an elastic member having an aperture.

3. The endoscope drying cabinet of claim 1 or claim 2, wherein the airflow accelerator is configured to operate at a plurality of different speeds to generate a plurality of first pressures and a plurality of second pressures, wherein the plurality of first pressures are greater than the plurality of second pressures.

4. The endoscope drying cabinet of any foregoing claim, wherein the airflow accelerator comprises a rotor.

5. A method for drying an endoscope, the method comprising:
receiving an endoscope drying cabinet comprising:
a first compartment configured to receive at least one endoscope;
a second compartment;
a partition separating the first and second compartments, wherein the partition comprises at least one aperture and an airflow accelerator configured to generate a first pressure in the first compartment and a second pressure in the second compartment, wherein the first pressure is greater than the second pressure;
a conduit extending through the at least one aperture, wherein a proximal end of the conduit terminates in the first compartment and a distal end of the conduit terminates in the second compartment; and
a connector comprising: a distal end coupled to the proximal end of the conduit and a proximal end configured to receive at least two different sizes of endoscope distal tips;
coupling a distal tip of an endoscope to the proximal end of the connector; and
activating the airflow accelerator to generate the first pressure in the first compartment and the second pressure in the second compartment.

6. The method of claim 5 , wherein the proximal end of the connector comprises an elastic member having an aperture, wherein the aperture has a first diameter, wherein the elastic member is configured to stretch to a second diameter that is between 5 percent greater than the first diameter and 50 percent greater than the first diameter and wherein coupling a distal tip of an endoscope to the proximal end of the connector comprises inserting the distal tip of the endoscope through the aperture of the elastic member.

7. The method of claim 5 or claim 6, further comprising a pressure indicator coupled to the first and second compartments, the pressure indicator configured to determine a difference between the first and second pressures, wherein activating the airflow accelerator to generate the first pressure in the first compartment and the second pressure in the second compartment comprises adjusting a speed of the airflow accelerator until a prescribed pressure is indicated on the pressure indicator.

8. An endoscope drying cabinet comprising:
a first compartment configured to operate at a first pressure;
a second compartment configured to operate at a second pressure, wherein the first pressure is greater than the second pressure;
a conduit extending between the first and second compartments, wherein a proximal end of the conduit terminates in the first compartment and a distal end of the conduit terminates in the second compartment, wherein the proximal end is configured to receive at least two different sizes of endoscope distal tips.

9. The endoscope drying cabinet of claim 8, wherein the proximal end comprises an elastic member having an aperture.

10. The endoscope drying cabinet of claim 2 or claim 9, wherein the aperture of the elastic member has a first diameter and wherein the elastic member is configured to stretch to a second diameter that is between 5 percent greater than the first diameter and 50 percent greater than the first diameter.

11. The endoscope drying cabinet of claim 10, wherein the elastic member is comprised of a polymeric substance.

12. The endoscope drying cabinet of any one of claims 1 to 4 or any one of claims 8 to 11, wherein the first compartment comprises an air exhaust aperture and the second compartment comprises an air intake aperture, optionally wherein the air intake aperture comprises a filter.

13. The endoscope drying cabinet of any one of claims 1 to 4 or any one of claims 8 to 12, wherein the second compartment comprises a moisture collector.

14. The endoscope drying cabinet of any one of claims 1 to 4 or any one of claims 8 to 13, further comprising a pressure indicator coupled to the first and second compartments, wherein pressure indicator is configured to determine a difference between the first and second pressures.

15. The endoscope drying cabinet of any one of claims 1 to 4 or any one of claims 8 to 14, wherein the first compartment is configured to operate at a plurality of first pressures and the second compartment is configured to operate at a plurality of second pressures, wherein the first pressures are greater than the second pressures.
